# EUROPEAN PATENT APPLICATION

(11) **EP 1 205 545 A1**
(43) Date of publication of application: **15.05.2002**
(21) Application number: 00953199.7
(22) Date of filing: 28.07.2000
(51) Int. Cl.: C12N 9/24, C12N 15/56, A61K 38/47, A01N 63/02

(54) **ENZYMES WITH BETA-(1,6)-ENDOGLUCANASE ACTIVITY**

(30) Priority: 31.07.1999 ES 9901747
(71) Applicant: NEWBIOTECHNIC, S.A., 41092 Sevilla (ES); Universidad de Sevilla, 41013 Sevilla (ES); Umiversidad De Salamanca, E-37008 Salamanca (ES)
(72) Inventor: MONTERO MACARRO, Manuel, E-41092 Sevilla (ES); REY BARRERA, Manuel, E-41092 Sevilla (ES); MONTE VAZQUEZ, Enrique, E-41092 Sevilla (ES); LLOBELL GONZALEZ, Antonio, E-41092 Sevilla (ES)
(86) International application number: ES0000293
(87) International publication number: WO0109295

(57) **Abstract**

This invention refers to enzymes with β-(1,6)-endoglucanase activity, to DNA constructs which encode said enzymes, to methods for the production of said enzymes, to enzymatic preparations which contain said enzymes and to the use of said enzymes and enzymatic preparations for the degradation or modification of materials containing β-(1,6)-glucan.

## Description

### FIELD OF THE INVENTION

This invention refers to enzymes with β-(1,6)-endoglucanase activity, to DNA constructs which encode said enzymes, to methods for the production of said enzymes, to enzymatic preparations which contain said enzymes and to the use of said enzymes and enzymatic preparations for the degradation or modification of materials which contain β-(1,6)-glucan.

### BACKGROUND OF THE INVENTION

There are organisms which have the ability of acting as biological control agents. Among these organisms there are certain species of fungi, for example, of the genus *Trichoderma,* which are useful as biological control agents against several phytopathogenic fungi. The degradation and subsequent assimilation of the phytopathogenic fungi, process known as mycoparasitism, has been proposed as the main mechanism to explain the antagonistic activity of said fungi against fungal pathogens. Mycoparasitism is a complex process which comprises several stages, including the penetration of the mycoparasite into the mycelium of the phytopathogenic fungus, which seems to be preceeded by an at least partial degradation of the cell wall of the phytopathogenic fungus.

The study of the expression models of the enzymes which degrade the cell wall of the fungi in different nutrient sources, and the biochemical characterisation of these enzymes, are essential prior requisites to understand the molecular basis of the antagonistic action of the biological control agents against phytopathogenic fungi. Upon this basis, the production of chitinases, β-1,6-glucanases, β-1,3-glucanases and extracellular proteases by different organisms, potentially useful as biological control agents when cultured in conditions which resemble said antagonism such as the presence of abundant polysaccharides in the cell walls of fungi (for example chitin), purified fungal cell walls or autoclave-sterilised mycelia as nutrient sources, has been analysed. Some of these hydrolases have been purified and are considered key enzymes in the first stages of antagonism.

Due to the complexity of fungal cell walls, hydrolytic activities with the ability to degrade other non-abundant cell wall polysaccharides are also interesting. Recent studies have shown that β-1,6-glucan is a key polymer in the structure of fungal cell walls, as it is involved in the binding of the major components which include β-1,6-glucan, chitin and mannoproteins. Consequently, enzymes which hydrolyse fungal β-1,6-glucans under the conditions previously mentioned, should contribute, together with the chitinases and the β-1,3-glucanases, to the effective rupture of fungal cell walls (phytopathogens, animal pathogens, food contaminants, etc.) during the antagonism.

The fungi of the genus *Trichoderma* have been since long studied both for their cellulolytic activity as well as for their antagonistic activity towards fungal plant pathogens. The antifungal mechanism of *Trichoderma* involves the use of enzymes which degrade the fungal cell wall, including chitinases, β-1,3- and β-1,6-glucanases and proteases. Due to the fact that chitin and β-1,3-glucan are the main structural components of fungal cell walls (except in the case of *Oomycetes*) it has been postulated that chitinases and β-1,3-glucanases are the key enzymes in the lysis of the cell walls of phytopathogenic fungi during the antagonistic action of *Trichoderma.* However, it seems that other cell wall-degrading enzymes, including those that hydrolyse the minoritary polymers (β-1,6-glucans, α-1,3-glucans, etc) can also be involved in the effective and complete degradation by *Trichoderma* of the cell walls of the mycelium and the conidia of phytopathogenic fungi.

β-(1,6)-endoglucanases (1,6,β-D-glucan glucano-hydrolases, EC 3.2.1.75) are enzymes which catalyse the cleavage of β-(1,6)-glucosidic bonds present in β-glucan, one of the cell wall components of many organisms, for example fungi and yeasts. These enzymes are also referred to as pustulanases due to their ability to degrade pustulan.

The presence of β-(1,6)-glucanase activity has been detected in several organisms.

Martin et al. [Applied and Environmental Microbiology, 1980, 40(6), 1136-1138] describe some *Bacillus* species which secrete β-(1,6)-glucanases.

Schep et al. [Biochem J., 1984, 223, 707-714] describe the purification and properties of a β-(1,6)-glucanase from *Penicillium brefeldianum.*

Dubourdieu et al. [Carbohydrate Research, 144, 277-287 (1985)] have described an industrial enzymatic preparation [NOVOZYM SP-116, of Novo Industri A/S] derived from a strain of *T. harzianum* which, among other glucanases, comprises a β-(1,6)-exoglucanase.

Pitson et al. [Enzyme Microbiol. Technol., 1993, 15, 178-192] describe that some filamentous fungi such *as Penicillium brefeldianum* or *Trichoderma harzianum* and yeasts such as *Saccharomyces cerevisiae* can produce enzymes which exhibit β-(1,6)-endoglucanase activity.

Mullenga et al. [Microbios, 1994, 80(234), 143-154] describe the isolation and characterisation of a β-(1,6)-glucanase from a strain of *Saccharomycopsis fibuligera.*

Extracellular β-(1,6)-glucanase activity has been described in a strain of *Trichoderma harzianum ,* described as an effective biological control agent, which grows on chitin as its single carbon source [de la Cruz et al., 1993, Arch. Microbiol., 159, 316-322]. Said activity is due to the presence of at least two β-(1,6)-glucanases known as BGN16.1 and BGN16.2 [De la Cruz et al., 1995, J. Bacteriol., 177, 1864-1871]. The enzyme BGN16.2 and the gene *bgn*16.2 have already been analysed [De la Cruz et al., 1995, J. Bacteriol., 177, 1864-1871; Lora et al., 1995, Mol. Gen. Genet., 247, 639-645]. However, enzyme BGN16.1 has not been purified or previously characterised, neither has the gene which encodes said enzyme, hence the structure of said enzyme, its relation with other enzymes or its evolutionary inferences is unknown.

The Unites States Patent US 5.770.406 describes an enzyme with β-1,6-endoglucanase activity derived from *Trichoderma harzianum* characterised in that it has an apparent molecular weight of 50 kDa determined by electrophoresis in denaturing conditions, at an optimum pH of 5, an optimum temperature comprised between 30°C and 40°C, with a Km of approximately 0.3% with respect to pustulan, with an apparent Ip of 5,6, a specific activity of approximately 100 U/mg of enzyme and an endo mode of action.

Although several enzymes with β-(1,6)-glucanase activity have been described in different organisms (bacteria, fungi, yeasts), the enormous variety which exists with regard to the mechanisms of action, substrate affinity, specificity, lytic ability, etc., still makes it necessary to increase the arsenal of enzymes with ability to degrade or modify the cell wall of deleterious organisms, for instance, phytopathogenic and saprophytic fungi, with the object of effectively being able to prevent the losses caused by said fungi.

The invention provides a solution to existing needs which comprises the purification and characterisation of enzymes with β-1,6-glucanase activity, the isolation and characterisation of the DNA sequences which code for said enzymes and the cloning of said DNA sequences.

The invention also provides a method for the production of said enzymes, enzymatic preparations containing said enzymes and the use of said enzymes and enzymatic preparations for the degradation or modification of materials which contain β-(1,6)-glucan.

### BRIEF DESCRIPTION OF DRAWINGS

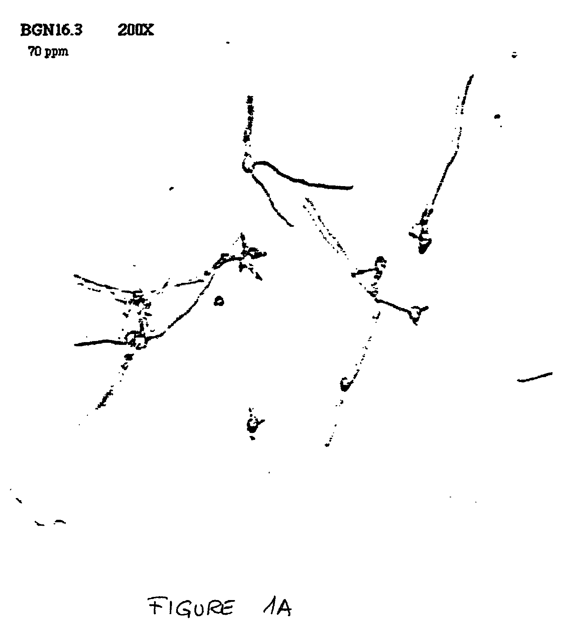

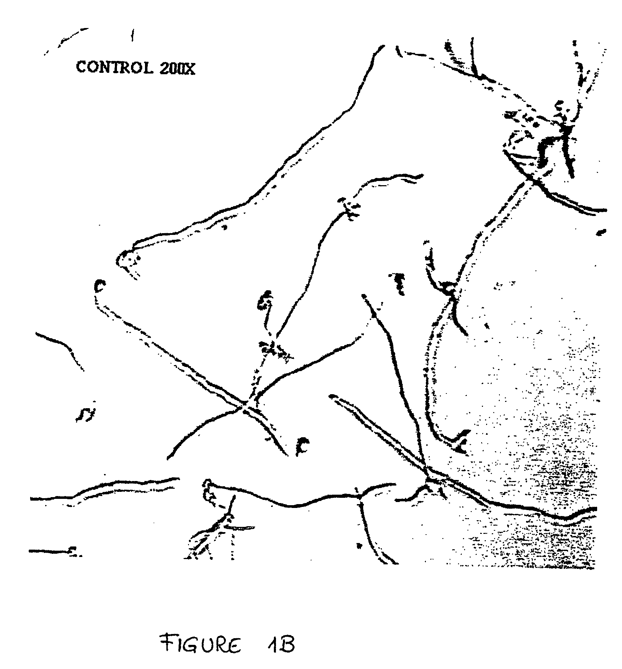

Figure 1 shows an image obtained with an optical microscope (200X amplification) in which it is possible to appreciate the antifungal effect, determined by the reduction in the size of the hyphae of the phytopathogenic fungus *Penicillium digitatum,* produced by the enzyme BGN16.1 [Figure 1A] and the effect of the negative control [Figure 1B].

### DETAILED DESCRIPTION OF THE INVENTION

The invention provides an enzyme with β-(1,6)-glucanase activity, from here on referred to as the enzyme of the invention, which has an amino acid sequence selected from:
a) The amino acid sequence shown in SEQ. ID. No.: 1,
b) The amino acid sequence shown in SEQ. ID. No.: 2, and
c) An amino acid sequence substantially homologous and functionally equivalent to the amino acid sequences shown in SEQ. ID. No.: 1 or in SEQ. ID. No.: 2.

In the sense used in this description, the expression "substantially homologous" means that the referred amino acid sequences have a degree of identity, at the amino acid level of at least 70%, preferably of at least 85% and more preferably of at least 95%.

Likewise, in the sense employed in this description, the expression "functionally equivalent" means that the referred protein has at least β-(1,6)-glucanase activity.

The enzyme the amino acid sequence of which is shown in SEQ. ID. No.: 1 is related to the enzyme known as BGN16.1, and consequently, it will be identified with the denomination BGN16.1 in this description. The existence of this enzyme has been previously proposed [De la Cruz et al., 1995, J. Bacteriol., 177, 1864-1871] although it has not been purified or characterised before, neither has the DNA sequence which encodes said enzyme, mostly due to technical difficulties posed by the very low expression levels which it exhibits [determined from the Northern blot assays and from the large number of clones screened to find a positive one].

The enzyme the amino acid sequence of which is shown in SEQ. ID. No.: 2 has been termed BGN16.3 in this description and has not been described previously.

The enzymes BGN16.1 and BGN16.3 present the characteristics which are summarised in Table 1.

**Table 1**

| **Properties of BGN16.1 and BGN16.3** | | |
|---|---|---|
| **PARAMETER** | **BGN16.1** | **BGN16.3** |
| **Molecular weight** | 51 KDa | 47,5 Kda |
| **(SDS)** | | |
| **Ip (IEF)** | 7,4 | 4,5 |
| **Ip (CF)** | 7,7 | 4,1 |
| **Inactivation T** | 50°C | 50°C |
| **Glycosilation** | No | No |
| **Optimum T** | 50°C | 50°C |
| **Km (pustulan)** | 0,08% | 0,11% |
| **Mode of action** | Endo | Endo |
| **Lysis rings in walls** | No | |
| **Specific activity** | 170 U/mg | 185 U/mg |

| | | |
|---|---|---|
| [SDS: Sodium dodecylsulphate: Ip: isoelectric point; | | |
| IEF: isoelectric focusing; CE: chromatofocusing] | | |

In the examples which accompany this description, the methods used to determine said properties are described in detail.

The enzyme of the invention can be obtained from an organism which produces it, such as a fungus of the genus *Trichoderma*, by a process which comprises culturing the producer organism under suitable conditions for the expression of said enzyme and, subsequently, recovering said enzyme. In a specific embodiment of this invention, the fungus employed belongs to the species *Trichoderma harzianum,* more specifically to the strain deposited in the Spanish Collection of Type Cultures (CECT) with the access number CECT 2413.

The culture of this producer organism can be carried out in two stages, as mentioned in Example 1. In the first stage spores of the fungus are inoculated into a culture medium supplemented with glucose as a carbon source and, subsequently, the mycelium is harvested, washed and cultured in suitable minimal medium supplemented with chitin as sole carbon source to induce the enzyme BGN16.1. Contrary to BGN16.1, BGN16.3 has the peculiarity of not being induced, or of doing it at undetectable amounts, by chitin, being only induced by the presence of the cell walls of fungi, for example, of *Botrytis*, or in the absence of a carbon source.

The β-(1,6)-glucanase activity can be assayed by means of the protocol described in Example 2.

The isolation and purification of the enzyme of the invention can be carried out by means of a process that comprises the precipitation of the enzyme with ammonium sulphate, the absorption-digestion to pustulan, the chromatofocusing of the dialysed solution coming from absorption-digestion to pustulan and gel filtration of the concentrated fractions that exhibited greatest β-(1,6)-glucanase activity (see Example 3).

Once isolated and purified, the enzyme of the invention was used in the physical-chemical and kinetic assays which are mentioned in Examples 4-11, the results of which are collected in Table 1 shown above. The antifungal activity of the enzyme of the invention is shown in Example 12.

With the enzyme of the invention as starting material, it is possible to identify and isolate the DNA sequence which codes for said enzyme by following a process which comprises:
- the creation of genomic DNA (gDNA) or copy DNA (cDNA) libraries of organisms which produce the enzyme of the invention;
- the sequencing of the amino-terminus of the enzyme of the invention and of tryptic fragments of the same;
- the design of suitable oligonucleotides to amplify, by means of the polymerase chain reaction (PCR), a region of the genomic clone of the organisms producers of the enzyme of the invention, that may serve to obtain probes for the screening of said libraries; and
- the analysis and selection of positive clones.

All these steps are described in greater detail in Example 13 which illustrates the obtaining of the DNA sequences which encode enzymes BGN16.1 and BGN16.3 of *T. harzianum.*

The extraction of gDNA can be carried out by means of a standard protocol [Kaiser et al., 1994, Methods in yeast genetics. A Cold Spring Harbour Laboratory Press, Cold Spring Harbour, New York] with some modifications, e.g. the addition of a purification step involving phenol [see Example 13.1].

For the creation of a cDNA library the total RNA is extracted from the organisms producers of the enzyme of the invention following a standard protocol [Chomczynski and Sacchi, 1987, Single-step method of RNA isolation by acid guanidinium thiocyanate-phenol-chloroform extraction. Anal. Biochem. 162:156-159] with slight modifications. In a specific embodiment, the producer organism of the enzyme of the invention is *T. harzianum* CECT 2413, which is cultured on a minimal medium with cell walls of organisms of the species *Botrytis* as sole carbon source, the messenger RNA (mRNA) being isolated, by oligo(dT)cellulose affinity chromatography. Subsequently, the cDNA is synthesised employing a commercial kit, suitable linkers are attached, it is ligated to a suitable vector and it is packaged into a suitable host, for example bacteriophage λ [see Example 13.2 and 13.3].

The sequencing of the amino-terminus of the enzyme and of tryptic fragments of the same can be carried out by any conventional procedure, for example using the Edmans Matsudaira method [A practical guide to protein and peptide purification for microsequencing. Academic Press, Inc. New York, Edmans Matsudaira (eds.) 1989] (see Example 13.4).

On the basis of the information obtained from the sequence of the amino-terminus and of the tryptic fragments of the enzyme of the invention, a set of oligonucleotides were designed for amplifying a specific sequence corresponding to the DNA sequence which encodes the enzyme of the invention. In a specific embodiment, the direct oligonucleotides were designed based on the sequences of the amino-terminal ends of the enzymes BGN16.1 and BGN16.3, whereas the reverse oligonucleotides (antisense) were designed from the sequences of the tryptic fragments of enzymes BGN16.1 and BGN16.3. Example 13.4 describes the sequences of the amino terminal ends and of the tryptic fragments of enzymes BGN16.1 and BGN16.3, which served to design the oligonucleotides used for performing the PCR [see Example 13.5].

The suitable fragments resulting from the amplification by PCR can be labelled and used as probes to screen a library (gDNA or cDNA) with the object of isolating the clones of interest by *in situ* hybridisation [see Example 13.6].

Therefore, the invention provides a DNA construct which encodes the enzyme of the invention which comprises:
a) a DNA sequence selected from SEQ. ID. No.: 3 and SEQ. ID. No.: 4.; or
b) a DNA sequence analogous to the sequence defined in a) that
   i. is substantially homologous to the DNA sequence defined in a); and/or
   ii. codes for a polypeptide that is substantially homologous to the protein encoded by the DNA sequence defined in a).

SEQ. ID. No.: 3 corresponds to the nucleotide sequence which codes for BGN16.1 whereas SEQ. ID. No.: 4 corresponds to the nucleotide sequence which codes for BGN16.3.

In the sense used in this description, the term "analogous" intends to include any DNA sequence which codes for an enzyme with β-1,6-endoglucanase activity which has the properties i)-ii) mentioned above. Typically the analogous DNA sequence:
- can be isolated from another organism which produces the enzyme with β-(1,6)-glucanase activity, on the basis of the DNA sequence shown in SEQ. ID, No.: 3 or SEQ. ID, No.: 4, or
- is constructed based on the DNA sequence shown in SEQ. ID, No.: 3 or in SEQ. ID, No.: 4, for example, by means of the introduction of conservative nucleotide substitutions, that is to say, that do not give rise to another amino acid sequence of the β-(1,6)-glucanase encoded by said DNA sequence shown in SEQ. ID, No.: 3 or in SEQ. ID, No.: 4, but which corresponds to codon utilisation of the host organism destined to the production of the enzyme, or rather by means of the introduction of nucleotide substitutions that give rise to a different amino acid sequence and, therefore, possibly, to a different protein structure that may give rise to a mutant β-(1,6)-glucanase with different properties to those of the wild-type enzyme. Other examples of possible modifications include the insertion of one or more nucleotides in the sequence, the addition of one or more nucleotides to any of the ends of the sequence, or the deletion of one or more nucleotides at any of the ends or within the interior of the sequence. For example, the analogous DNA sequence can be a sub-sequence of the DNA sequence shown in any of the sequences shown in SEQ. ID, No.: 3 or in SEQ. ID, No.: 4.

Generally, the analogous DNA sequence is substantially homologous to the DNA sequence encoding a β-(1,6)-glucanase enzyme of the invention, for example SEQ. ID, No.: 3 or SEQ. ID, No.: 4., i.e. it exhibits a homology at the nucleotide level of at least 70%, preferably at least 85% or more preferably at least 95% with respect to any of the sequences mentioned above.

The DNA sequence which codes for the enzyme of the invention may originate not only from *T. harzianum* CECT 2413, but also from any other strain of *Trichoderma* or from a host organism transformed with said DNA sequence.

Alternatively, the DNA sequence which encodes the enzyme of the invention, can be isolated, by conventional techniques, from the DNA of any organism by the use of probes or of oligonucleotides prepared on the basis of the information regarding the DNA sequence provided in this description, or by means of primers (by PCR).

The DNA sequence which codes for the enzyme of the invention, or the construct that contains it, can be inserted into an appropriate vector. Therefore, the invention also refers to a vector, such as an expression vector, which comprises said DNA sequence, or a construct that contains it. The choice of vector will be dependant on the host cell in which it is to be subsequently introduced. By way of example, the vector into which said DNA sequence is to be introduced may be a plasmid or a vector that, when it is introduced into the host cell, becomes integrated in the genome of said cells and replicates together with the chromosome or chromosomes in which it has integrated.

In the vector provided by this invention, the DNA sequence which encodes the enzyme of the invention must be operationally connected to a promoter and to a termination sequence. The promoter may be any DNA sequence which exhibits transcriptional activity in the chosen host cell and may derive in genes that code for homologous or heterologous proteins of the host cell. The procedures used to ligate the DNA sequence which encodes the enzyme of the invention to the promoter and to the terminator sequence, respectively, and to insert said construct into a vector are well known in the art and have been described, for example, in Sambrook et al. [Molecular Cloning. A Laboratory Manual, Cold Spring Harbor, NY, 1989].

The invention also provides a cell which comprises the DNA sequence which encodes the enzyme of the invention, or a DNA construct which contains said sequence or said vector mentioned above. The host cells that can be transformed with the DNA sequence which encodes the enzyme of the invention can be prokaryotic or, preferably, eukaryotic cells, such as cells from plant tissues or fungal cells, for example yeast cells or filamentous fungi cells. The transformation of these cells can be carried out by means of conventional techniques, such as for example, by techniques which involve the formation of protoplasts and their transformation followed by the regeneration of the cell wall. The transformation of plant tissue derived cells can be very interesting from several points of view, for example, to increase the resistance to phytopathogenic fungi.

The invention also provides a method for the production of an enzyme of the invention, which comprises culturing a suitable host cell which contains the DNA sequence which encodes the enzyme of the invention, under conditions which permit the production of the enzyme and the recovery of the enzyme in the culture medium.

The medium used to culture the transformed host cells can be any medium suitable to culture the referred host cells. The β-(1,6)-glucanase expressed can be, advantageously, secreted to the culture medium and can be recovered by a procedure as that described in Example 3 or by any other conventional protein isolation procedure which comprises the separation of the cells from the culture medium, the precipitation of the proteins and their separation by chromatographic methods.

The invention also provides an enzymatic preparation useful for the degradation or modification of materials which contain β-(1,6)-glucan, for example, the material of the microbial cell wall, which at least comprises an enzyme with β-(1,6)-glucanase activity of the invention. Said enzymatic preparation may contain between 0.01% and 100% by weight of said enzyme with β-(1,6)-glucanase activity of the invention.

In a specific embodiment, the enzymatic preparation provided by this invention is a single-component enzyme preparation which essentially contains one or more of the enzymes with β-(1,6)-glucanase activity of the invention.

In another specific embodiment, the enzymatic preparation provided by the invention comprises multiple enzyme activities, for example, different enzyme activities required for the modification or the degradation of microbial cell walls. By way of example said enzymatic preparation may contain lytic enzymes, particularly of microbial origin (fungal or bacterial), for example, derived from different species of the genus *Trichoderma, Oerskovia, Arthrobacter, Rhizotocnia, Staphylococcus* or *Streptomyces,* It may also contain one or more enzymes capable of modifying or degrading cell walls, for example, enzymes with cellulolytic, mannanolytic, chitinolytic or proteolytic activity, such as cellulases, other β-(1,6)-glucanases, β-(1,3)-glucanases, mannanases, endo- or exo- chitinases, chitosanases, proteases, α- or β-mannosidases, mutanases, etc. These enzymes may originate from any producer organism, such as several species of the genus *Aspergillus* or those mentioned above in relation to lytic enzymes.

The enzymatic preparation of the invention may be prepared by conventional methods and can be presented in liquid or in solid form, for example, in the form of a granulate. The enzymatic preparation may contain additives, for example, stabilisers which extend the stability of the latter.

The invention also provides an antifungal preparation which comprises, at least, an enzyme of the invention together with, at least, a chemical fungicide. The chemical fungicide employed can be any of those in common use, preferably, a chemical fungicide selected from the group of chemical fungicides which affect the membrane, chemical fungicides which affect the synthesis of cell walls and mixtures thereof. Optionally, the antifungal composition of the invention may contain, additionally, at least, a protein with microbial cell wall-degrading enzymatic activity. Any enzyme with activity to degrade the bacterial cell wall may be used, if so desired, in the antifungal composition of the invention.

The antifungal composition of the invention, which may contain between 0.01% and 99.99% by weight of the enzyme of the invention, may be prepared by conventional methods and can be presented in liquid or in solid form, for example, in the form of a granulate. The antifungal preparation of the invention may also contain additives, for example, stabilisers which extend the stability of the latter.

The enzyme of the invention may be used to degrade or modify materials which contain β-(1,6)-glucan, for example, microbial cell walls. In a preferred specific embodiment, the enzyme of the invention can be used as a biofungicide against several deleterious organisms, for instance, against phytopathogenic organisms (including fungi which damage crops and fungi which contaminate fruits before and after harvesting), fungal animal pathogens (including fungal human pathogens), fungi contaminating food, surfaces and equipment, and, generally, any fungus which produces economic losses in any industrial, agricultural or livestock sector.

The enzyme of the invention can also be used to break or lyse the cell wall of different microorganisms to recover products of interest which are produced by said microorganisms. In this sense it is remarkable that BGN16.1 is approximately 10-fold more effective in breaking or lysing the cell wall of *Saccharomyces cerevisiae* than the enzyme described in United States Patent US 5.770.406.

The enzymatic preparation of the invention can be designed at will to have a composition specifically adapted to the cell wall that is to be broken or lysed. If the cell wall to break contains a protein-mannane complex and a glucan, the enzymatic preparation could contain advantageously, a mixture of protease, mannanase, chitinase and β-glucanase activities, with the object of effectively breaking said cell wall.

Other applications of the enzyme of the invention include, by way of illustration, but are not limited to, the extraction of mannoproteins from the external layer of yeast cell walls; the production of protoplasts from yeasts or fungi; the preparation of yeast or fungal extracts; the improvement of filtration steps in processes for the production of wines, must and juices; the elaboration of wines with altered organoleptic properties by overexpression of the gene if wine-yeasts; the elaboration of compositions for washing teeth and dentures; the elimination of fungi from coatings; the treatment of textiles, for example, the removal of excess colouring from cloths; the elaboration of compositions for removing dental plaque; the elaboration of compositions for the removal of biological films (biofilms) deposited on surfaces, for example, on the surface of a contact lens.

The enzyme or the enzymatic preparation or the antifungal composition provided by the invention can be used in the control of deleterious organisms, for example, against phytopathogenic fungi (including fungi which damage crops and fungi which contaminate fruits before and after harvesting), fungal animal pathogens (including human fungal pathogens), fungi contaminating food, surfaces and equipment, and, generally, any fungus which produces economic losses in any industrial, agricultural or livestock sector. In the sense used in this description, the term "control" includes the reduction or arrest of the growth and/or germination that may result in the elimination of said deleterious organisms or in the reduction of the damages caused by them. Therefore, in a specific embodiment of the invention, said enzymatic preparations or antifungal compositions will be pharmaceutical compositions or compositions for their application in the agricultural sector.

The enzyme, enzymatic preparation or antifungal composition provided by the invention can also be used to disinfect, prevent and/or treat the infection caused by fungal animal pathogens in livestock installations, which are prone to being infected by fungal animal pathogens which grow and thrive upon substrates which are in contact with the animals, for example, the straw used in animal bedding, or the housing conditions of animals, which involve a risk of said animals being infected by said pathogens.

On the other hand, as is well known, on occasions, test samples for analysis, for example biological samples, food, etc., present fungal contamination which make the analysis of said samples more difficult or impossible. The invention provides a solution to said problem, consisting in the use of an enzyme, an enzymatic preparation or an antifungal composition of the invention to control the fungal contamination in said test samples for analysis, by its application onto the samples.

The antifungal composition of the invention may be used to control all types of deleterious fungi, such as those mentioned previously, by the control mechanism known as integrated management.

The dosage of the enzymatic preparation and of the antifungal composition provided by the invention and their usage conditions can be determined by methods known in the art.

The following examples serve to illustrate the present invention and must not be regarded as limiting of the scope thereof.

### EXAMPLE 1

### T. harzianum culture

*T*. *harzianum* CECT 2413 was obtained from the Spanish Type Culture Collection, CECT (Valencia, Spain) and was maintained in PPG medium (mashed potato + glucose, both at 2%), supplemented with 2% agar.

In order to produce and purify the enzymes BGN16.1 and BGN16.3, as well as to produce the cDNA library, *T. harzianum* was cultured in two stages, following protocols already described by De la Cruz et al. [J. Bacteriol., 1995., Vol. 177, No. 7, 1864-1871].

Briefly, to produce BGN16.1, in a first stage, spores of *T. harzianum* are inoculated at a final concentration of 10⁸ spores/ml in minimal medium (MM) [KH₂PO₄, 15 g/l; (NH₄)₂SO₄, 5 g/l; 1 ml/l of oligoelements [FeSO₄ · 7H₂O, 5 g/l; MnSO₄ · H₂O, 1.6 g/l; ZnSO₄ · 7H₂O, 1.4 g/l; CoCl₂ · 6H₂O, 3.7 g/l)] supplemented with 2% glucose as carbon source. The final pH of the culture medium is adjusted to 5.5 with 1 M KOH. *T. harzianum* is cultured in this pre-induction medium for 48 h at 28 °C with orbital shaking at 200 rpm. Subsequently the mycelium is harvested, successively washed with MgCl₂ and H₂O and it is transferred to MM supplemented with 1.5% chitin as carbon source.

A similar process to that described for the production of BGN16.1 was followed for the production of BGN16.3, but using Czapek's medium [MgSO₄·7H₂O, 0.5 g/l; FeSO₄ ·7H₂O, 0.01 g/l; KCl 0.425 g/l; MgCl·6H₂O, 0.115 g/l; NH₄Cl, 2.1 g/l; NaH₂PO₄, 0.92 g/l] as pre-induction medium and without adding chitin to the induction medium as BGN16.3 has the peculiarity of not being induced by chitin (at least at detectable amounts) but rather by fungal cell walls, for example *Botrytis cinerea*, or in the absence of a carbon source, preferably by fungal cell walls.

### EXAMPLE 2

### β-(1,6)-glucanase activity assay

The β-(1,6)-glucanase activity is determined by means of an assay which consists in preparing a test mixture containing 0.8 ml of pustulan solution [β-(1,6)-glucan] at 0.5% (w/v) in 50 mM, pH 5.5, potassium acetate buffer, and 0.2 ml of the enzymatic solution suitably diluted in the same buffer. The reaction mixture is incubated at 37 °C for a time period comprised between 30 minutes and 1 hour, stopping the reaction by heating it to 100°C for 7 minutes. Subsequently the increase in the amount of reducing sugars is determined in 0.15 ml of the reaction mixture by the method of Somogyi (J. Biol. Chem., 1952, 195, 19-23) and Nelson [Methods Enzymol., 1957, 3, 85-86], using glucose as a standard at concentrations comprised between 0 and 1 mg/ml. Enzyme and substrate blanks are included.

One unit of β-(1,6)-glucanase activity is defined as the amount of enzyme that liberates 1 µmol of reducing sugar equivalents, expressed as glucose, per minute, under the conditions assayed.

The protein concentration is determined by the method of Bradford (Anal. Biochem., 1976, 72, 248-254).

### EXAMPLE 3

### Purification of enzymes BGN16.1 and BGN16.3

The following process, composed of the following stages, was followed for the purification of enzymes BGN16.1 and BGN16.3:

### a) Precipitation with ammonium sulphate

This step, as all others (unless indicated otherwise) was carried out at 4 °C. Cultures of *T. harzianum* incubated for 48h in MM supplemented with 1.5% chitin (for the production of BGN16.1) or in Czapek medium supplemented with *Botrytis* cell walls at 0.1% (for the production of BGN16.3) were filtered through Whatman No. 1 filter paper and were centrifuged at 6000 g for 10 minutes. Subsequently, the supernatant (about 200 ml) was precipitated with ammonium sulphate at 80% saturation. After overnight incubation, the precipitate was recovered by centrifuging at 12.000 g for 20 minutes, it was resuspended in the smallest possible volume of distilled water and was dialysed against 50 mM, pH 5,5, potassium acetate buffer.

### c) Adsorption-digestion to pustulan

For the adsorption to pustulan (*Umbilicaria papullosa,* Calbiochem) 3 ml aliquots of the dialysed supernatant were incubated with 0.6 ml of particulate pustulan with ethanol following the procedure described by De la Cruz et al., [J. Bacteriol., 1995, 177, 1864-1871]. The incubation was carried out for 20 minutes with magnetic stirring and the mixture was subsequently centrifuged at 12.000 g for 10 minutes. The supernatant (the fraction not adsorbed to pustulan) is incubated again with pustulan (the process is repeated two more times). The precipitates obtained after the successive adsorption steps are washed three times with 3 ml of a 1M NaCl solution in phosphate buffer-KOH 70 mM, pH 6.0 and, finally, it is resuspended in 50 mM, pH 5.5, potassium acetate buffer with 1 mM phenylmethyl-sulphonyl fluoride and 1 mM sodium azide. These samples are incubated overnight at 37 °C. The clarified solution resulting from the degradation of pustulan is centrifuged at 12.000 g for 10 minutes. The supernatant (5-10 ml) is dialysed again against a 25 mM, pH 7.4, imidazole-HCl buffer.

### c) Chromatofocusing

The dialysed solution from stage b) is subjected to chromatofocusing. In order to do this a calibrated glass column, model C10/20 (1 cm diameter and 20 cm height) of Pharmacia (Sweden), containing a bed of 18 ml of Polybuffer Exchanger 94 of the same brand, limited on its upper end by 1 ml of Sephadex G-25 (Pharmacia) and an AC10 plunger, is packed following the procedure described by the manufacturer. The chromatography is carried out at 4 °C. The column is equilibrated with 20 volumes of imidazole-HCl buffer. Polybuffer 74 (Pharmacia) diluted 1:8 in water was used as eluent. The solution was previously degassed and the pH was adjusted to 4.0. A flow of 9 ml/h was applied by means of an LKB 10200 peristaltic pump connected at the exit of the column and fractions of approximately 1.5 ml were collected using a Pharmacia Frac-100 automatic collector. After measuring the pH of the different fractions, the β-(1,6)-glucanase activity was assayed. The fractions which exhibited the greatest activity were concentrated down to approximately 0.5 ml using a Centricon 10 concentrator (Amicon, Beverley, Mass.).

### d) Gel filtration

The set of concentrated BGN16.1 fractions is applied to a column (1.6 x 40 cm) of Sephacryl S-200 HR (Pharmacia) equilibrated with 100 mM potassium acetate buffer, pH 5.5 with 100 mM HCl, and is eluted with the same buffer at a flow of 7 ml/h. Again, the fractions are assayed to determine the β-(1,6)-glucanase activity, selecting those which exhibit the highest levels, which are then collected, washed and concentrated in 50 mM potassium acetate buffer, pH 5.5, using a Centricon 10 concentrator. The purified proteins are stored at 4 °C.

The purification of BGN16.3 was carried out by gel filtration in an FPLC with a Protein Pack 125 (Waters) column. After introducing the sample, a 0.1 M KCl solution in 50 mM potassium acetate buffer, pH 5.5, was applied at a flow of 0.2 ml/min. Fractions were collected every 60 seconds (0.2 ml/fraction). The appearance of the protein was monitored by means of an absorbance detector adjusted at 280 nm.

### EXAMPLE 4

### Determination of the molecular weight of enzymes BGN16.1 and BGN16.3

The molecular weight of the enzymes BGN16.1 and BGN16.3 was determined by analytical electrophoresis under denaturing conditions and by gel filtration chromatography.

### 4.1. SDS-PAGE

The electrophoresis of the enzymes BGN16.1 and BGN16.3, under denaturing conditions (electrophoresis in a polyacrylamide gel treated with sodium dodecylsulphate, SDS-PAGE) was carried out according to the procedure described by Weber and Osborn (1975) [The Proteins (Vol. 1), pag. 179-221, Neurath and Hill (eds.), Academic Press, Inc., New York], using 12% acrylamide-bisacrylamide gels and a Mini-Protean II apparatus of Bio-Rad (USA) and following the manufacturer's instructions.

The apparent molecular weight of the enzymes BGN16.1 and BGN16.3, under the conditions assayed, is of 51 kDa and of 47-48 kDa, respectively.

### 4.2. Gel Filtration

The molecular weight of the native BGN16.1 enzyme was determined by a gel filtration technique using a Sephacryl S-200 HR column (Pharmacia) (1.6 x 40 cm) calibrated with marker proteins, yielding a value of 20-25 kDa for BGN16.1 and of approximately 28 kDa for BGN16.3.

### EXAMPLE 5

### Determination of the isoelectric point of the enzymes BGN16.1 and BGN16.3

In order to determine the isoelectric point (Ip) of the enzymes BGN16.1 and BGN16.3, isoelectric focusing techniques and chromatofocusing (CF) techniques have been employed.

Isoelectric focusing was carried out following the procedure described by Robertson et al. [Anal. Biochem., 1987, 167, 290-294]. The proteins were visualised by Coomassie Blue staining. The apparent Ip value for the enzymes BGN16.1 and BGN16.3 was calculated by reference to conventional marker proteins with Ip values comprised between 3.5 and 9.3 (Amersham-Pharmacia). The apparent Ip values calculated by isoelectric focusing were of 7.4 for BGN16.1 and of 4.5 for BGN16.3.

By using acid chromatofocusing, an Ip value of 4.1 was calculated for BGN16.3, and, as a result of alkaline chromatofocusing, an Ip value of 7.7 was calculated for BGN16.1.

### EXAMPLE 6

### Effect of temperature on the stability and activity of enzymes BGN16.1 and BGN16.3

### 6.1. Inactivation temperature

In order to know the stability of the purified enzymes BGN16.1 and BGN16.3 versus temperature, the purified proteins were incubated for 30 minutes (BNG16.1) or for 5 or 10 minutes (BGN16.3) at temperatures comprised between 20°C and 80°C in 50 mM potassium acetate buffer, pH 5.5 and, subsequently, the remaining activity was measured at 37°C by adding pustulan (5 mg/ml) as substrate for the assay. The inactivation temperature, which is defined as the temperature at which the specific activity is reduced by 50% in the conditions described, is of 50°C for both BGN16.1 and BGN16.3.

### 6.2. Optimum temperature

The optimum temperatures of enzymes BGN16.1 and BGN16.3 were determined by conventional tests in which the enzyme activity was assessed at a temperature interval comprised between 20°C and 80°C. The optimum temperature obtained was of 50°C, at pH 5.5, for BGN16.1, that is, the same as the inactivation temperature, which seems to suggest that pustulan stabilises the enzyme. Likewise, the optimum temperature obtained for BGN16.3 was of 50°C at pH 5.5.

### EXAMPLE 7

### Optimum pH

In order to determine the optimum pH for enzyme BGN16.3, conventional assays were carried out using pustulan as substrate and buffering the assay at different pH values. For pH values comprised between 3 and 5, 50 mM citrate-acetic acid buffer was used, whereas for pH values comprised between 6 and 8, 50 mM phosphate buffer was used, and for pH 9, 50 mM Tris-HCl buffer was employed. The maximum activity of enzyme BGN16.3 was observed at pH 5.

### EXAMPLE 8

### Substrate specificity

In order to study the specificity of purified BGN16.1 and BGN16.3 towards different substrates, their enzyme activities were assayed against several substrates, which had α- or β-glycosidic bonds, at a concentration of 5 mg/ml. When glucans were used, the reaction products were determined by using the conventional tests described previously. Chitinase and chitosanase activities were determined according to the procedure described by De la Cruz [Eur. J. Biochem., 1992, 206,856-867].

The hydrolysis of the substrates by the purified enzymes BGN16.1 and BGN16.3 was carried out by incubating 4 mg of pustulan or 2 mg of gentibiose (a β-1,6-disaccharide) with 2 µg of purified protein in 1 ml of distilled water, at different times, at 37°C. Substrate blanks were run in parallel. After the hydrolysis, the reactions were stopped by heating them to 100°C for 5 minutes and the pustulan oligomers were analysed by high pressure liquid chromatography (HPLC) using an HPX 42-A column maintained at 60°C. Water was used as eluent at a flow velocity of 0.6 ml/min. The products were detected based on their absorbance at 195 nm and were identified by comparison with glucose, gentibiose and cellulose oligosaccharide standards (oligomerisation velocity of 2 to 5).

Table 2 contains the different substrates assayed and shows the results obtained in percentage with respect to the maximum activity reached in each case.

**Table 2**

| **Specificity of BGN16.1 and BGN16.3 towards different substrates** | | | |
|---|---|---|---|
| **Substrates** | **Main bond** | **BGN16.1** | **BGN16.3** |
| Laminarin | β-1,3: β-1,6(Glc) | 22 | 8 |
| Pachyman | β-1,3 (Glc) | 0 | 0 |
| Pustulan | β-1,6(Glc) | 100 | 100 |
| Glucan(*S. cerevisiae*) | β-1,3: β-1,6(Glc) | 73 | 18 |
| Carboxymethylcellulose | β-1,4(Glc) | 0 | 0 |
| Colloidal chitin | β-1,4(GlcNAc) | 0 | 0 |
| Glycol-chitosan | β-1,4(GlcN) | 0 | NP |
| Nigeran | α-1,3: α-1,4(Glc) | 0 | 0 |
| Soluble starch | α-1,4: α-1,6(Glc) | 0 | 0 |
| Dextran | α-1,6(Glc) | 0 | NP |

| | | | |
|---|---|---|---|
| [NP: Not performed] | | | |

Pustulan (from *Umbilicalia papullosa*) and pachyman (from *Poria coccos*) were from Calbiochem (La Jolla, CA). Carboxymethylcellulose, chitin (from crab shells), dextran (from *Leuconostoc mesenteroides*), glycol-chitosan, laminarin (from *Laminaria digitata*), nigeran (from *Aspergillus nidulans*) and the soluble starch were acquired from Sigma Chemical Co. (St. Louis, MO). Yeast glucan was prepared from baker's yeast according to the methodology described by Rombouts and Phaff [Eur. J. Biochem., 1976, 63, 109-120].

As can be appreciated, the activity of the enzyme BGN16.1 is specific for its substrate (pustulan) [β-1,6-glucan], acts to a lesser extent on yeast glucan [β-1,3: β-1,6 glucan (4:1)] and displays reduced activity towards laminarin, a polysaccharide formed essentially by β-(1,3)-glucan which has approximately 15% β-1,6-glycosidic type bonds, which would be those that would become hydrolysed. No activity was detected in the other substrates tested.

The results shown in Table 2 also evidence that the activity of enzyme BGN16.3 is specific towards pustulan (substrate) and shows a very reduced activity towards yeast glucan and laminarin, no activity being detected against the other substrates tested.

### EXAMPLE 9

### Michaelis-Menten Constants

In order to study the affinity of BGN16.1 and BGN16.3 enzymes towards its substrates, assays were carried out employing different concentrations of pustulan. The Michaelis-Menten constants (Km) were determined according to the Lineweaver-Burk plot from the data obtained measuring the initial velocity of the hydrolysis of pustulan and using a pustulan concentration interval of 20 to 0.5 mg/ml. The initial velocities of hydrolysis of pustulan were calculated measuring the activity under the test conditions described above at different times of 0 and 30 minutes.

The results obtained were:
BGN16.1
   - Km: 0,8 mg pustulan/ml
   - Vmax: 312 µmol of product/minute· mg BGN16.1
BGN16.3
   - Km: 1,1 mg pustulan/ml
   - Vmax: 391 µmol of product/minute· mg BGN16.3

### EXAMPLE 10

### Reaction products and action mechanisms

In order to determine whether the mechanism of action of the enzymes BGN16.1 and BGN16.3 is of the exo- or endo-hydrolytic type an assay was carried out which consisted in performing an HPLC analysis of the products released at different times of the hydrolysis of pustulan by said purified enzymes.

Briefly, 100 µl of a reaction mixture which comprises pustulan and the enzyme (BGN16.1 or BGN16.3) the mechanism of action of which is to be studied, were injected into an Aminex HPX 42A column maintained at 45°C, using water as the eluent. The refraction index of each of the products was used as the basis for their detection. The reaction products were identified by comparing their elution times with those of glucose and cellulose oligosaccharide (degree of polymerisation 2 to 4) standards.

### BGN16.1

The results obtained evidenced that incubation of purified BGN16.1 with pustulan generated a series of oligosaccharides of smaller molecular weight which hydrolysed to even smaller oligosaccharides when the enzyme incubation time was increased. Specifically, the analysis revealed an initial release of long oligosaccharides which became excised to smaller saccharides, which oscillated from glucose (Glc) to gentitetraose (Glc4), being gentibiose (Glc2) the major end-product of the hydrolysis of pustulan by BGN16.1. When gentibiose is used as substrate, the dissacharide was not hydrolysed by the enzyme BGN16.1. Consequently, these results indicate that purified BGN16.1 has an endohydrolytic mechanism of action.

### BGN16.3

The results obtained evidenced that incubation of purified BGN16.3 with pustulan generated a very small amount of glucose and a high amount of oligosaccharides with different degrees of polymerisation, which indicated that the mechanism of action of purified BGN16.3 is endohydrolytic. This was confirmed when observing the inability of BGN16.3 to degrade gentibiose, a dimer of β-(1,6) glucan which would only be susceptible of degradation by exo-β-(1,6) glucanases. The profile obtained in the degradation products of pustulan by the action of BGN16.3 is different to that obtained with other β-(1,6) glucanases.

### EXAMPLE 11

### Hydrolysis of fungal cell walls

The ability of purified enzymes BGN16.1 and BGN16.3 to degrade the cell walls of different fungi has been studied. This was done by incubating the purified (0,5 U) enzymes (BGN16.1 in some cases and BGN16.3 in others) with 4 mg of lyophilised cell walls of different fungi in 1 ml assay in 50 mM potassium acetate buffer, pH 5.5.

The fungi tested to study the ability of purified BGN16.1 were *Botrytis cineres* CECT 2100, *Giberella fujikuroi* IMI 58289, *Phytophthora syringae* CECT 2351 and *Saccharomyces cerevisiae* (baker's yeast "La Cinta Roja®", Spain). The mixtures were incubated at 37°C for 16 hours with sporadic agitation. The reactions were stopped by centrifugation (10.000 x g, 10 minutes) and the amount of reducing sugars liberated in the supernatants was determined by the method described by Somogyi [J. Biol. Chem., 1952, 195, 19-23] and Nelson [Methods Enzymol., 1957, 3, 85-86]. Enzyme and substrate blanks were included. The results obtained are shown in Table 3 and demonstrate that BGN16.1 only exerts enzymatic activity upon the cell walls of *S*. *cerevisiae,* whereas BGN16.3 does not exert enzymatic activity on any of the cell walls tested.

**Table 3**

| **Lytic action of BGN16.1 and BGN16.3 upon cell walls** | | |
|---|---|---|
| **Cell walls** | **BGN16.1 (%)** | **BGN16.3 (%)** |
| *B. cinerea* CECT 2100 | 0 | 0 |
| *G. fujikuroi* IMI 58289 | 0 | 0 |
| *P. syringae* CECT 2351 | 0 | 0 |
| *S. cerevisiae* | 20 | 0 |

The lytic activity upon cell walls was also determined by observing the clearance rings in agar plates which contained the previously mentioned fungal cell walls (final concentration 1 mg/ml), using the procedure described by Tanaka and Phaff [J. Bacteriol., 1965, 89, 1570-1580]. After 48 hours of incubation at 37°C, the plates were stained according to the procedure described by Grenier et al. [Plant Physiol., 1993, 103, 1277-1283] with 0.005% (w/v) aniline blue, and were washed with water. The hydrolytic rings were observed under ultraviolet light and the radii of the largest rings were measured. The results obtained evidence that BGN16.1 did not produce clearance rings upon the cells walls of filamentous fungi or yeasts. However, when BGN16.1 was combined with other glucanases [BGN13.1 and BGN16.2] of *T. harzianum* [De la Cruz, J. Bacteriol., 1995, 177, 1864-1987], both clearance rings as well as antifungal activity against phytopathogenic fungi was observed, which points to a possible synergistic effect in the association of enzymes with different lytic activities.

### EXAMPLE 12

### Antifungal activity assay

In order to determine the antifungal activity of the enzymes of the invention, the following assay was carried out. In the well of a microplate, 3000 spores of *Penicillium digitatum* are grown in PDA (Potato Dextrose Agar, Difco), diluted three-fold with respect to the concentration recommended by the manufacturer, together with the enzyme the antifungal effect of which is to be investigated. The plate is maintained 18 h at 25°C and is subsequently observed under the microscope. As negative control, an assay is carried out using only PDB and spores. Both germination inhibition of the spores as well as the decrease in size of the hyphae of *Penicillium digitatum* is analysed.

For the enzyme BGN16.3, it has been observed that at a concentration of 70 µg/ml a 50% reduction in the size of the hyphae is obtained with respect to the control, no inhibitory effect being observed upon the germination of the spores.

Figure 1A shows the antifungal effect produced by the enzyme BGN16.3, at a concentration of 70 µg/ml (70 ppm), on 3000 spores of *Penicillium digitatum* after 18 hours of incubation. Figure 1B shows the result of a negative control that contained only PDA and spores. Figure 1 clearly shows the size reduction of the hyphae of *Penicillium digitatum* due to the antifungal action of the enzyme BGN16.3.

### EXAMPLE 13

### Cloning of the DNA sequences that encode the enzymes BGN16.1 and BGN16.3

In order to obtain the complete cDNA clone of the enzymes BGN16.1 and BGN16.3 a strategy was followed that comprised the use of PCR. To do this, degenerate oligonucleotides were designed based on the microsequencing of the amino terminus and of tryptic peptides of the enzymes BGN16.1 and BGN16.3.

For the design of the direct oligonucleotides, the sequences of the amino termini of the enzymes BGN16.1 and BGN16.3 were used, whereas in the design of the reverse oligonucleotides, the sequences of the internal peptides (tryptic fragments) were employed.

Using said oligonucleotides and under the experimental conditions which are described below (see Example 13.5) specific bands were obtained of the coding sequences of BGN16.1 and BGN16.3 respectively, which were subcloned into a pGEM-T vector. The sequencing of the amplified bands confirmed that these were fragments of the clones sought. Subsequently, the fragments obtained by PCR were radioactively labelled and were employed as probes to screen a cDNA library of *T. harzianum* CECT 2413. Of the total number of clones screened some positive clones were obtained, the analysis of which evidenced that they carried inserts that completely contained the ORF and part of the flanking sequences of the promoter and terminator regions.

### 13.1 Extraction of gDNA

The gDNA of *Trichoderma harzianum* CECT 2413 was obtained according to the protocol described by Kaiser et al. (1994) [Methods in yeast genetics. A Cold Spring Harbour Laboratory Press, Cold Spring Harbour, New York] with some modifications, including the addition of a purification step using phenol. Briefly, 0.3-0.5 g of lyophilised mycelium is resuspended in 50 mM Tris-HCl and 20 mM EDTA, it is homogenised and 200 ml of 10% sodium dodecylsulphate (SDS) are added. Subsequently the mixture is incubated at 65°C for 30 minutes, sodium acetate is added and it is incubated for 30 more minutes at 4°C. Finally the mixture is centrifuged and the supernatant is treated with phenol and precipitated with ethanol.

### 13.2 Extraction of total RNA

The total RNA of *T. harzianum* CECT 2413 was extracted using the acid phenol method described by Chomczynski and Sacchi (1987) [Single-step method of RNA isolation by acid guanidinium thiocyanate-phenol-chloroform extraction. Anal. Biochem., 162:156] with minor modifications.

### 13.3 Construction of a cDNA library

Using the total RNA of *T. harzianum* CECT 2413 as starting material, obtained after culturing said fungus in MM with Botrytis cell walls at 0.5% as carbon source for 9 hours, the mRNA was isolated. In order to achieve this, oligo(dT)cellulose affinity chromatography (Stratagene, La Jolla, CA) was used. Subsequently, the cDNA was synthesised using a commercial kit "ZAP-cDNA synthesis kit" (Stratagene). Eco RI and Xho I linkers were bound to the cDNA so obtained and was ligated to the vector Uni-Zap XR (Stratagene). Finally, the packaging kit "Gigapack III gold packaging extract" (Stratagene) was used to package the ligated cDNA into phage λ, following the instructions of the manufacturer.

### 13.4 Microsequencing of the proteins

Microsequencing was carried out following the method of Edmans Matsudaira [A practical guide to protein and peptide purification for microsequencing. Academic Press, Inc. New York, Edmans Matsudeira (eds.) 1989] in order to determine the amino terminus of the enzymes BGN16.1 and BGN16.3, as well as to obtain internal fragments that would allow the cloning of the corresponding gene.
The results obtained were as follows:
BGN16.1: Amino terminus: ASSFASSQDGRYQFTAAQAP [SEQ. ID. No.: 5] Internal peptide: KVKGKLIGGGRGNNKLNHY [SEQ. ID. No.: 6]
BGN16.3:

Amino terminus: AAGAQAYASNQAGN [SEQ. ID. No.: 7]

Internal peptide: GLNSNLQIFGSPW [SEQ. ID. No.: 8]

### 13.5 Polymerase Chain Reaction (PCR)

Degenerate oligonucleotides were designed based on partial sequences of enzymes BGN16.1 and BGN16.3 for the PCR experiments.

In the case of BGN16.1, the direct and reverse oligonucleotides were: direct: 5′-GATGGNCGNTAYCARTTYACNGC-3′ [SEQ. ID. No.: 9] reverse: 5′-CCDATNARYTTNCCYTTNACYTT-3′ [SEQ. ID. No.: 10] where
N is inosine;
Y is T or C;
R is G or A; and
D is A or G or T

In the case of BGN16.3, the direct and reverse oligonucleotides were: direct: 5'-GCTGGNGCNCARGCN TAYGC-3' [SEQ. ID. No.: 11] reverse: 5′-CCANGGNCTNCCRAANATYTG-3′ [SEQ. ID. No.: 12] where
N is inosine;
Y is T or C;
R is G or A; and
D is A or G or T

The following parameters were used for carrying out the PCR reactions:
- 1 denaturation cycle at 95°C for 1 minute followed by
- 35 synthesis cycles composed by:
   - a hybridisation phase at:
      i) 55°C for 1 minute (for the sequence which codes for BGN16.1), or
      ii) 50°C for 1 minute (for the sequence which codes for BGN16.3),
- an elongation phase at 72°C for 1 minute, and
- a denaturation phase at 95°C for 1 minute; and finally
- 1 additional elongation cycle of 7 minutes at 72°C to complete unfinished products.

In all cases the PCR was performed using gDNA as template and using 100 pmoles of each oligonucleotide, for a total PCR volume of 25 µl.

### 13.6. Screening of the cDNA library

The screening of the cDNA library was carried out following conventional methods described by Sambrook et al. [Molecular Cloning. A Laboratory Manual, Cold Spring Harbour, NY, 1989], using probes labelled radioactively with α³²P-dCTP following the random primer method described by Feingerg and Vogelstein (1983) in Anal. Biochem., 132:6. In order to do this the commercial kit "Oligolabelling kit" [Pharmacia, Sweden] was used following the manufacturer's instructions.

### 13.7 Cloning of the cDNA corresponding to the enzyme BGN16.1

The sequence of the amino terminus of BGN16.1 [SEQ. ID. No.: 5] was used for the design of the direct oligonucleotide, and for the design of the reverse oligonucleotide the sequence of an internal peptide was used [SEQ. ID. No.: 6].

Using said oligonucleotides and under the experimental conditions previously described (see example 13.5) a specific band of 455 nucleotides was obtained which was subcloned into the commercial vector pGEM-T (Promega, WI). Subsequently, the sequencing of said band confirmed that it was a fragment of the clone sought. Next, the fragment obtained by PCR was radioactively labelled and was used as a probe to screen a λ Zap library constructed from the RNA extracted from *T. harzianum* CECT 2413 after 9 hours of induction in MM with *Botrytis* cell walls. Of approximately 400.000 clones screened, two positive clones were obtained (Zbgn1.I and ZBgn1.II). For the analysis of said clones, firstly the phage was excised following the manufacturer's instructions, proceeding later to the sequencing of both strands of the inserts it carried. The two sequenced clones carried truncated clones of 1,4 kbp which contained the complete ORF except for the signal peptide and a fragment of the non-coding 3' region.

### 13.8 Cloning of the cDNA corresponding to the enzyme BGN16.3

The sequence of the amino terminus of BGN16.3 [SEQ. ID. No.: 7] was used for the design of the direct oligonucleotide, and for the design of the reverse oligonucleotide the sequence of an internal peptide was used [SEQ. ID. No.: 8].

Using said oligonucleotides and under the experimental conditions previously described (see example 13.5) a specific band of 450 nucleotides was obtained which was subcloned into the commercial vector pGEM-T (Promega, WI). Subsequently, the sequencing of said band confirmed that it was a fragment of the clone sought. Next, the fragment obtained by PCR was radioactively labelled and was used as a probe to screen a λ Zap library constructed from the RNA extracted from *T. harzianum* CECT 2413 after 9 hours of induction in MM with *Botrytis* cell walls. Of approximately 400.000 clones screened, two positive clones were obtained (ZBgn3.I and ZBgn3.II). For the analysis of said clones, firstly the phage was excised following the manufacturer's instructions, and the phagemid so obtained was sequenced from both strands. In both cases the vectors carried a 1,7 kbp insert which contained the complete ORF and part of the flanking sequence of the promoter and terminator region.

### EXAMPLE 14

### Southern blot analysis

To determine the presence of the gene that encodes BGN16.3 in other strains and species of *Trichoderma* other *than T*. *harzianum* CECT 2413 (from which the clone was initially cloned), a Southern blot analysis was carried out with genomic DNA of different species of the genus *Trichoderma* identified by their sequence ITS 1-2, more specifically,. *T. harzianum* (IMI 296235), *T. reesei* (IMI 113135), *T. asperellum* (IMI 20179), *T. asperellum* (IMI 20268), *T. koningii,* T. *inhamatum* (IMI 352940), *T. longibrachiatum* (CECT 2414), *T. longibrachiatum* (IMI 304058), *T. pseudokoningii, T. atroviride* (IMI 352941) and *T. atroviride* (IMI 352939). *T. harzianum* (CECT 2413) was used as control.

High stringency hybridisation conditions were used (65°C) with a probe that included all the cDNA of BGN16.3 of *T. harzianum* CECT 2413 radioactively labelled by the random primer technique using the "Oligolabelling kit" (Pharmacia). Between 5-10 µg of DNA extracted by conventional methods and digested with EcoRI were loaded.

The results obtained permit to observe a hybridisation signal of the BGN16.3.probe in all the *Trichoderma* strains analysed, which indicates the presence of the gene which codes for BGN16.3, with minimum variations, in all those strains.

### Example 15

### Regulation of the expression of BGN16.3 by Western Blot

In order to know the conditions in which BGN16.3 is produced in *T. harzianum,* inductions were performed under different conditions and the presence of said enzyme was studied with specific polyclonal antibodies against it obtained in New Zealand rabbits.

Each of the lanes of the gel were loaded with 15 µg of extracellular protein originating from each of the induction conditions employed, more specifically:

| Lane | Induction conditions |
|---|---|
| 1 | 2% glucose |
| 2 | 2% glycerol |
| 3 | 1.5% chitin |
| 4 | cell walls (*B. cynerea*) at 0.5% |
| 5 | 0.5% pustulan |
| 6 | absence of carbon |
| 7 | pure BGN16.3 of *T. harzianum* |

A standard Western blot protocol was used for the detection of BGN16.3, using the primary antibody at a 1:1000 dilution and using anti-rabbit IgG conjugated with horseradish peroxidase (Sigma Chemical Co.) diluted 1:2000 as the secondary antibody.

The maximum production of the protein BGN16.3 was observed in the inductions carried out with cell walls (*Botrytis cynerea*) at 0.5% and in the induction with pustulan, also appearing in the induction in conditions of absence of a carbon source.

LIST OF SEQUENCES

(1) GENERAL INFORMATION:
(i) APPLICANT:
(A) NAME: NEWBIOTECHNIC, S.A.
(B) STREET: Avda. Américo Vespucio, 69
(C) CITY: Sevilla
(D) PROVINCE: Sevilla
(E) COUNTRY: Spain
(F) POSTAL CODE (ZIP): 41092
(G) TELEPHONE: 954 081 031
(H) FAX: 954 081 034
(i) APPLICANT:
(A) NAME: UNIVERSIDAD DE SEVILLA
(B) STREET: Valparaíso, 5
(C) CITY: Sevilla
(D) PROVINCE: Sevilla
(E) COUNTRY: Spain
(F) POSTAL CODE (ZIP): 41013
(G) TELEPHONE: 954 237 871
(H) FAX: 954 238 537
(i) APPLICANT:
(A) NAME: UNIVERSIDAD DE SALAMANCA
(B) STREET: Patio de Escuelas, 1
(C) CITY: Salamanca
(D) PROVINCE: Salamanca
(E) COUNTRY: Spain
(F) POSTAL CODE (ZIP): 37008
(G) TELEPHONE: 923 294 416
(H) FAX: 923 294 418
(ii) TITLE OF THE INVENTION:
ENZYMES WITH BETA-(1,6)-ENDOGLUCANASE ACTIVITY
(iii) NUMBER OF SEQUENCES: 12
(iv) COMPUTER READABLE FORM:
(A) TYPE OF SUPPORT: Floppy disk
(B) COMPUTER: IBM PC compatible
(C) OPERATIVE SYSTEM: PC-DOS/MS-DOS
(D) SOFTWARE: PatentIn Release #1.0, Version #1.30 (OEP)

(2) INFORMATION OF SEQ. ID. No. 1:

(i) SEQUENCE CHARACTERISTICS:
(A) LENGTH: 452 amino acids
(B) TYPE: amino acid
(C) STRANDNESS: unknown
(D) TOPOLOGY: unknown

(ii) TYPE OF MOLECULE: Protein

(xi) DESCRIPTION OF SEQUENCE SEQ. ID. No. 1:

Asp Gly Arg Tyr Gln Phe Thr Ala Ala Gln Ala Pro Val Leu Gly Ala
1               5                   10                  15
Gly Asn Pro Gly Ile Gln Asp Trp Gln Leu Phe Ile Lys Glu Lys Ser
            20                  25                  30
Gly Arg Lys Gln Thr Val Arg Gly Phe Gly Ala Ala Ile Thr Asp Ala
        35                  40                  45
Thr Val Ala Ala Phe Asn Lys Leu Asn Ala Asn Ser Arg Thr Gln Leu
    50                  55                  60
Phe Asn Asp Leu Met Thr Pro Ser Gly Leu Asn Phe Asn Leu Leu Arg
65                  70                  75                  80
His Thr Val Ala Ser Ser Asp Leu Ser Ala Asp Leu Ala Tyr Thr Tyr
                85                  90                  95
Asp Asp Ala Gly Gly Asn Val Asp Thr Gly Leu Asn Ser Phe Gly Leu
            100                 105                 110
Gly Asp Arg Gly Asn Ala Met Ile Ser Met Leu Ala Asn Phe Arg Arg
        115                 120                 125
Leu Gln Pro Gln Leu Thr Ile Val Gly Ser Pro Trp Ser Ala Pro Gly
    130                 135                 140
Trp Met Lys Val Lys Gly Lys Leu Ile Gly Gly Gly Thr Gly Asn Asn
145                 150                 155                 160
Lys Leu Asn His Ala Tyr Glu Asn Ala Tyr Ala Gln Tyr Phe Val Lys
                165                 170                 175
Tyr Leu Gln Ala Tyr Glu Ala Gly Gly Ala His Ile Asp Ala Ile Thr
            180                 185                 190
Leu Gln Asn Glu Pro Leu Asn Asn Lys Asp Asp Met Pro Thr Met Gln
        195                 200                 205

Ile Glu Ala Ala Glu Ser Gly Ala Leu Ile Arg Asp Lys Val Gly Pro
    210                 215                 220
Ala Leu Arg Asn Ala Gly Leu Asn Thr Gln Ile Trp Ala Trp Asp His
225                 230                 235                 240
Asn Gln Asp Val Tyr Ser Tyr Pro Gln Thr Val Met Asn Thr Ala Ser
245 · 250 255
Gln Tyr Val Gln Ala Ala Ala Trp His Cys Tyr Ala Gly Asn Ala Pro
260 265 270
Glu Asn Trp Thr Pro Leu Thr Gln Phe His Asn Glu Phe Pro Gly Lys
275 280 285
Glu Gln Tyr Met Thr Glu Cys Trp Thr Ser Val Leu Ser Gly Gly Thr
290 295 300
Asp Trp Val His Lys Leu Gln Leu Arg Pro Val Pro Pro Pro Asn Trp
305 310 315 320
Ala Asn Gly Ile Ile Ala Trp Thr Leu Gly Thr Ser Thr Ala Ala Gly
325 330 335
Leu Arg Phe Leu Val Ala Val Thr Ala Thr Thr Ala Leu Val Leu Leu
340 345 350
Pro Ser Thr Leu Met Val Thr Gly Thr Lys Arg Arg Leu Thr Thr Thr
355 360 365
Met Leu Gly Gln Phe Ser Arg Tyr Ile Leu Lys Gly Ala Val Val Val
370 375 380
Asp Gly Thr Gly Ser Trp Leu Phe Asp Pro Asn Thr Gly Val Glu Ser
385 390 395 400
Val Ala Thr Val Asn Pro Asp Gly Thr Arg Thr Val Val Ile Gln Asn
405 410 415
Arg Tyr Asn Asn Asp Ile Trp Val Arg Leu Ala Thr Glu Ser Glu Ser
420 425 430
Gln Thr Trp Asn Ala Arg Val Pro Ala Arg Ala Val Thr Thr Trp Ile
435 440 445
Leu Pro Lys Ala
450

(2) INFORMATION OF SEQ. ID. No. 2:

(i) SEQUENCE CHARACTERISTICS:
(i) SEQUENCE CHARACTERISTICS:
(A) LENGTH: 490 amino acids

(B) TYPE: amino acid
(C) STRANDNESS: unknown
(D) TOPOLOGY: unknown

(ii) TYPE OF MOLECULE: Protein

(xi) DESCRIPTION OF SEQUENCE SEQ. ID. No. 2:

Met Arg Tyr Ala Leu Ile Ala Ser Met Leu Gly Gln Ala Ala Ile Ser
1               5                   10                  15
Val Ala Met Pro Ser Glu Pro Ala His Ser Pro Arg Ala Ala Gly Ala
            20                  25                  30
Gln Ala Tyr Ala Ser Asn Gln Ala Gly Asn Tyr Lys Leu Thr Ser Ile
        35                  40                  45
Ala Ala Pro Val Gln Gly Asn Gly Ser Pro Gly Pro Ser Thr Trp Asn
    50                  55                  60
Leu Ser Ile Asp Asp Thr Ser Ser Gly Tyr Lys Gln Lys Ile Val Gly
65                  70                  75                  80
Phe Gly Ala Ala Val Thr Asp Ala Thr Val Ser Ala Phe Asn Glu Leu
                85                  90                  95
Ser Ala Ser Thr Leu Ser Gln Leu Leu Asp Glu Leu Met Thr Gly Ala
            100                 105                 110
Gly Ala Ser Phe Ser Leu Met Arg His Thr Ile Gly Ala Ser Asp Leu
        115                 120                 125
Ser Gly Asp Pro Ala Tyr Thr Tyr Asp Asp Asn Gly Gly Asn Ala Asp
    130                 135                 140
Pro Gly Met Thr Gly Phe Asn Leu Gly Asp Arg Gly Thr Ala Met Ala
145                 150                 155                 160
Thr Met Leu Ala Gln Met Lys Gly Leu Asn Ser Asn Leu Gln Ile Phe
                165                 170                 175
Gly Ser Pro Trp Ser Ala Pro Gly Trp Met Lys Leu Asn Asn Ala Ile
            180                 185                 190
Asp Gly Asn Thr Asn Asn Asn Asn Leu Asn Asp Gly Tyr Leu Thr Asn
        195                 200                 205
Asn Gly Ala Gln Tyr Ser Ala Ala Phe Ala Gln Tyr Phe Val Lys Tyr
    210                 215                 220
Ile Gln Ala Phe Glu Ser His Gly Ala Thr Ile Asn Ala Ile Thr Leu
225                 230                 235                 240

Gln Asn Glu Pro Leu Asn Ser Gln Ala Gly Tyr Pro Thr Met Tyr Met
                245                 250                 255
Phe Ser Tyr Glu Gln Gly Asp Leu Ile Gln Asn Tyr Val Ala Pro Ala
            260                 265                 270
Leu Lys Ala Ala Gly Leu Ser Thr Lys Ile Trp Ala Tyr Asp His Asn
        275                 280                 285
Thr Asp Gln Pro Asp Phe Pro Glu Gln Val Met Gly Ile Ala Ala Asp
    290                 295                 300
Asp Val Ser Ala Val Ala Trp His Cys Tyr Ala Thr Asn Leu Asp Trp
305                 310                 315                 320
Thr Val Leu Thr Asn Phe His Asn Ser Tyr Pro Asn Thr Asp Gln Tyr
                325                 330                 335
Met Thr Glu Cys Trp Thr Pro Ser Thr Gly Ala Trp Asn Gln Ala Ala
            340                 345                 350
Ser Phe Thr Met Gly Pro Leu Gln Asn Trp Ala Arg Gly Val Ala Ala
        355                 360                 365
Trp Thr Leu Gly Thr Thr Ala Gln Asp Gly Pro His Leu Ser Ser Gly
    370                 375                 380
Gly Cys Gly Thr Cys Thr Gly Leu Val Thr Ile Asn Asn Gly Gln Tyr
385                 390                 395                 400
Thr Phe Gln Thr Ala Tyr Tyr Met Met Ala Gln Phe Ser Lys Phe Met
                405                 410                 415
Pro Val Gly Ala Thr Val Leu Ser Gly Thr Gly Ser Tyr Thr Tyr Ser
            420                 425                 430
Gly Ser Gly Gly Val Gln Ser Val Ala Ser Leu Asn Pro Asp Gly Thr
        435                 440                 445
Arg Thr Val Val Ile Glu Asn Thr Phe Gly Asn Asp Ile Tyr Ile His
    450                 455                 460
Leu Ser Thr Ser Ser Gly Gln Glu Trp Ser Gly Asn Val Pro Thr Asn
465                 470                 475                 480
Ser Val Thr Thr Trp Val Leu Pro Ala Val
                485                 490

(2) INFORMATION OF SEQ. ID. No. 3:

(i) SEQUENCE CHARACTERISTICS:
(A) LENGTH: 1528 bases
(B) TYPE: nucleic acid

(C) STRANDNESS: single
(D) TOPOLOGY: unknown

(ii) TYPE OF MOLECULE: DNA

(xi) DESCRIPTION OF SEQUENCE SEQ. ID. No. 3:

GATGGGCGGT ACCAGTTCAC GGCCGCCCAA GCTCCTGTCC TGGGTGCCGG CAACCCTGGC 60
ATCCAAGACT GGCAGCTGTT CATCAAGGAG AAGTCCGGAC GCAAGCAGAC CGTCAGGGGT 120
TTTGGTGCTG CCATCACCGA TGCCACCGTC GCCGCCTTCA ACAAGCTCAA CGCAAACTCT 180
CGCACGCAGC TGTTCAACGA CCTGATGACC CCTTCGGGCC TCAACTTCAA CCTGCTGCGT 240
CACACCGTCG CTAGCTCGGA TCTCTCTGCT GATCTCGCCT ACACTTACGA CGACGCCGGT 300
GGTAATGTCG ACACTGGTCT CAACAGCTTT GGCCTGGGTG ATCGTGGAAA TGCCATGATC 360
AGCATGCTGG CCAACTTCCG CCGCCTGCAG CCCCAGCTGA CCATTGTCGG CTCGCCTTGG 420
TCTGCACCGG GATGGATGAA GGTCAAGGGC AAGCTGATTG GCGGCGGCAC GGGCAACAAC 480
AAGCTCAACC ACGCGTACGA GAACGCCTAC GCCCAGTACT TTGTCAAGTA CCTCCAGGCG 540
TACGAGGCTG GCGGTGCTCA CATTGACGCC ATCACTTTGC AGAACGAGCC CCTGAACAAC 600
AAGGACGATA TGCCTACCAT GCAGATCGAG GCGGCGGAGT CTGGTGCTTT GATCCGCGAC 660
AAGGTTGGCC CTGCTCTGCG CAATGCCGGC CTCAACACCC AGATCTGGGC TTGGGACCAC 720
AACCAGGATG TGTACTCTTA CCCCCAGACT GTCATGAACA CGGCCAGCCA GTACGTCCAG 780
GCCGCTGCTT GGCACTGCTA CGCCGGCAAC GCCCCTGAGA ACTGGACTCC TCTGACCCAG 840
TTCCACAACG AGTTCCCCGG CAAGGAGCAG TACATGACCG AGTGCTGGAC CTCCGTCCTC 900
TCCGGCGGCA CCGACTGGGT CCACAAGCTC CAGCTTCGCC CTGTTCCCCC TCCAAACTGG 960
GCCAACGGCA TCATCGCCTG GACTCTGGGT ACTTCAACGG CGGCGGGCCT GCGCTTTCTG 1020
GTGGCGGTAA CTGCCACAAC TGCACTGGTC CTGTTACCGT CAACGCTGAT GGTAACGGGT 1080
ACAAAAAGGA GATTGACTAC TACAATGCTG GGGCAATTCT CGCGCTACAT CCTAAAGGGC 1140
GCTGTTGTTG TTGATGGTAC TGGTAGCTGG TTGTTTGATC CCAACACTGG TGTTGAGAGT 1200
GTTGCTACTG TTAACCCTGA TGGCACCCGC ACGGTCGTCA TTCAGAACCG ATACAACAAT 1260
GATATTTGGG TTCGTTTGGC TACGGAGTCG GAGAGCCAGA CTTGGAACGC GCGTGTTCCT 1320
GCTCGGGCTG TGACTACTTG GATCCTGCCC AAGGCTTAAG GCGGAGAACA TGGTATGTGA 1380
TGAAATGATG AATTTGAAAT GAAGATGGGA TGGGTGGTGG AAGCACTGTA TATATCTACC 1440
TAGCTTCGTC TGTATATAGA CAACTTGAGT ACATGTAACT GTCTAAAATG AATATACATG 1500
TATAAATGGT AAAAAAAAAA AAAAAAAA 1528

(2) INFORMATION OF SEQ. ID. No. 4:

(i) SEQUENCE CHARACTERISTICS:
(A) LENGTH: 16961528 bases
(B) TYPE: nucleic acid
(C) STRANDNESS: single
(D) TOPOLOGY: linear

(ii) TYPE OF MOLECULE: DNA

(xi) DESCRIPTION OF SEQUENCE SEQ. ID. No. 4:

TGAAGATCGA CAGCTCAAGC TCTTCAAAGG TTGTAAGCTA CAAGTCAAAG ACACTTTCAA 60
GTTCCCAAAC CGCCAAAATG CGTTACGCTC TCATCGCCTC CATGCTTGGC CAAGCAGCCA 120
TCAGCGTTGC TATGCCTTCG GAGCCCGCTC ACAGCCCTCG TGCTGCCGGT GCTCAGGCCT 180
ACGCATCCAA CCAGGCCGGT AACTACAAGC TCACCTCCAT TGCTGCTCCC GTTCAAGGCA 240
ATGGAAGCCC TGGCCCATCT ACTTGGAACC TGTCCATCGA CGACACCAGC AGTGGTTACA 300
AGCAGAAGAT CGTCGGTTTC GGTGCTGCCG TGACTGATGC CACCGTCTCT GCCTTCAACG 360
AGCTGTCTGC CAGCACCCTC AGCCAGCTGC TCGACGAGCT CATGACTGGT GCTGGTGCCA 420
GCTTCTCTCT CATGCGCCAC ACCATCGGTG CCTCTGACTT GTCTGGCGAC CCAGCCTACA 480
CTTATGACGA CAACGGCGGC AACGCTGACC CTGGCATGAC TGGCTTCAAC CTCGGAGACC 540
GTGGTACTGC CATGGCCACC ATGCTGGCTC AGATGAAGGG CCTCAACTCC AACCTCCAGA 600
TCTTTGGATC TCCCTGGAGT GCTCCTGGAT GGATGAAGCT GAACAACGCC ATTGACGGCA 660
ACACCAACAA CAACAACCTG AACGATGGTT ACCTCACCAA CAACGGTGCT CAGTACTCGG 720
CCGCTTTTGC CCAGTACTTT GTCAAGTACA TCCAGGCATT CGAGTCGCAC GGCGCCACCA 780
TCAATGCCAT TACCCTCCAG AACGAGCCCC TGAACAGCCA GGCTGGCTAT CCCACCATGT 840
ACATGTTCTC GTACGAGCAG GGAGACCTGA TCCAGAACTA CGTCGCCCCT GCCTTGAAGG 900
CGGCCGGTCT GAGCACCAAG ATCTGGGCTT ACGACCACAA CACTGACCAA CCTGATTTCC 960
CCGAGCAGGT CATGGGCATC GCCGCAGACG ATGTCTCTGC CGTTGCATGG CACTGCTATG 1020
CCACTAACCT GGACTGGACT GTCCTGACCA ACTTCCACAA CTCCTACCCC AACACTGACC 1080
AGTACATGAC TGAGTGCTGG ACCCCCTCCA CTGGTGCTTG GAACCAGGCC GCCTCCTTCA 1140
CCATGGGCCC TCTCCAGAAC TGGGCCCGTG GTGTTGCTGC ATGGACCCTC GGCACCACTG 1200
CCCAGGACGG CCCTCACCTG TCCAGCGGTG GCTGCGGCAC TTGCACTGGT CTCGTCACCA 1260
TCAACAACGG CCAGTACACC TTCCAGACTG CTTACTACAT GATGGCTCAG TTCTCCAAGT 1320
TCATGCCCGT TGGAGCCACC GTCCTGAGCG GCACTGGCAG CTACACTTAC TCCGGCAGCG 1380
GCGGTGTTCA GTCTGTTGCT TCTCTCAACC CCGATGGCAC CCGCACGGTC GTCATCGAGA 1440
ACACCTTCGG CAACGACATT TACATCCACC TGAGCACCTC CAGTGGCCAG GAGTGGAGCG 1500
GCAACGTCCC TACCAACTCT GTCACCACCT GGGTCCTCCC CGCTGTTTAA GCAGATTACG 1560
GATTGTTGGA GATGAAGGAA CCTTTGATGT AAATATTTGT CTACATGTGG ATTTGCCACT 1620
GCACATACGT ATCTGGGGAT TATTTACCAT GCTGTAGAAT GAATGAATAC GGTTAGCCTA 1680
AAAAAAAAAA AAAAAA 1696

(2) INFORMATION OF SEQ. ID. No. 5:

(i) SEQUENCE CHARACTERISTICS:
(A) LENGTH: 20 amino acids
(B) TYPE: amino acid
(C) STRANDNESS: unknown
(D) TOPOLOGY: unknown

(ii) TYPE OF MOLECULE: Peptide

(v) TYPE OF FRAGMENT: N-terminal

(xi) DESCRIPTION OF SEQUENCE SEQ. ID. No. 5:

Ala Ser Ser Phe Ala Ser Ser Gln Asp Gly Arg Tyr Gln Phe Thr Ala
1               5                   10                  15
Ala Gln Ala Pro
            20

(2) INFORMATION OF SEQ. ID. No. 6:

(i) SEQUENCE CHARACTERISTICS:
(A) LENGTH: 19 amino acids
(B) TYPE: amino acid
(C) STRANDNESS: unknown
(D) TOPOLOGY: unknown

(ii) TYPE OF MOLECULE: Peptide

(iii) TYPE OF FRAGMENT: Internal

(xi) DESCRIPTION OF SEQUENCE SEQ. ID. No. 6:

Lys Val Lys Gly Lys Leu Ile Gly Gly Gly Arg Gly Asn Asn Lys Leu
1               5                   10                  15
Asn His Tyr

(2) INFORMATION OF SEQ. ID. No. 7:

(i) SEQUENCE CHARACTERISTICS
(A) LENGTH: 14 amino acids
(B) TYPE: amino acid
(C) STRANDNESS: unknown
(D) TOPOLOGY: unknown

(ii) TYPE OF MOLECULE: Peptide

(v) TYPE OF FRAGMENT: N-terminal

(xi) DESCRIPTION OF SEQUENCE SEQ. ID. No. 7:

Ala Ala Gly Ala Gln Ala Tyr Ala Ser Asn Gln Ala Gly Asn
1               5                   10

(2) INFORMATION OF SEQ. ID. No. 8:

(i)  SEQUENCE CHARACTERISTICS
(A) LENGTH: 134 amino acids
(B) TYPE: amino acid
(C) STRANDNESS: unknown
(D) TOPOLOGY: unknown

(ii) TYPE OF MOLECULE: Peptide

(v) TYPE OF FRAGMENT: Internal

(xi) DESCRIPTION OF SEQUENCE SEQ. ID. No. 8:

Gly Leu Asn Ser Asn Leu Gln Ile Phe Gly Ser Pro Trp
1               5                   10

(2) INFORMATION OF SEQ. ID. No. 9:

(i) SEQUENCE CHARACTERISTICS:
(A) LENGTH: 23 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 9:

GATGGNCGNT AYCARTTYAC NGC                                    23

[N = Inosine]

(2) INFORMATION OF SEQ. ID. No. 10:

(i) SEQUENCE CHARACTERISTICS:
(A) LENGTH: 23 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 10:

CCDATNARYT TNCCYTTNAC YTT 23

[N = Inosine]

(2) INFORMATION OF SEQ. ID. No. 11:

(i) SEQUENCE CHARACTERISTICS:
(A) LENGTH: 20 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 11:

GCTGGNGCNC ARGCNTAYGC 20

[N = Inosine]

(2) INFORMATION OF SEQ. ID. No. 12:

(i) SEQUENCE CHARACTERISTICS:
(A) LENGTH: 21 bases
(B) TYPE: nucleic acid
(C) STRANDNESS: single
(D) TOPOLOGY: linear

(ii) TYPE OF MOLECULE: Other DNA
(A) DESCRIPTION: /desc: "synthetic"

(xi) DESCRIPTION OF SEQUENCE SEQ. ID. No. 12:

CCANGGNCTN CCRAANATYT G 21

[N = Inosine]

## Claims

1. An enzyme with β-(1,6)-endoglucanase activity **characterised in that** it has an amino acid sequence selected from:
a) the amino acid sequence shown in SEQ. ID. No.: 1,
b) the amino acid sequence shown in SEQ. ID. No.: 2, and
c) an amino acid sequence substantially homologous and functionally equivalent to the amino acid sequences shown in SEQ. ID. No.: 1 or in SEQ. ID. No.: 2.

2. An enzyme according to claim 1, **characterised in that** it has the amino acid sequence shown in SEQ. ID. No.: 1.

3. An enzyme according to claim 2, **characterised in that** it has an apparent molecular weight determined in denaturing conditions of approximately 51 kDa.

4. An enzyme according to claim 2, **characterised in that** it has an apparent isoelectric point of 7.4, determined by isoelectric focusing.

5. An enzyme according to claim 2, **characterised in that** it has an optimum temperature of approximately 50°C.

6. An enzyme according to claim 2, **characterised in that** the Km for pustulan is approximately 0.08%.

7. An enzyme according to claim 2, **characterised in that** it has an endohydrolytic mechanism of action.

8. An enzyme according to claim 1, **characterised in that** it has the amino acid sequence shown in SEQ. ID. No.: 2.

9. An enzyme according to claim 8, **characterised in that** it has an apparent molecular weight determined in denaturing conditions of approximately 47-48 kDa.

10. An enzyme according to claim 8, **characterised in that** it has an apparent isoelectric point of 4.5, determined by isoelectric focusing.

11. An enzyme according to claim 8, **characterised in that** it has an optimum temperature of approximately 50°C.

12. An enzyme according to claim 8, **characterised in that** the Km for pustulan is approximately 0.11%.

13. An enzyme according to claim 8, **characterised in that** it has an endohydrolytic mechanism of action.

14. A sequence of isolated DNA which comprises a DNA sequence which encodes an enzyme according to any of claims 1 to 13, or a fragment thereof.

15. A DNA sequence according to claim 14, **characterised in that** is has a nucleotide sequence selected from:
a) SEQ. ID. No.: 3,
b) SEQ. ID. No.: 4, and
c) a DNA sequence analogous to the sequences defined in a) and in b) that (i) is substantially homologous to the DNA sequence defined in a) or in b); and/or (ii) encodes a polypeptide that is substantially homologous to the protein encoded by the DNA sequences defined in a) or in b).

16. A recombinant vector **characterised in that** it contains a DNA sequence according to any of claims 14 or 15.

17. A cell **characterised in that** it contains a DNA sequence according to any of claims 14 or 15, or a vector according to claim 16.

18. A method for the production of an enzyme according to any of claims 1 to 13, which comprises culturing a cell according, to claim 17 under conditions that allow the production of the enzyme and the recovery of the enzyme from the culture medium.

19. An enzymatic preparation useful for the degradation or modification of materials that contain β-(1,6)-glucan, which comprises at least an enzyme according to any of claims 1 to 13.

20. Enzymatic preparation according to claim 19, which comprises between 0.01% and 100% by weight of an enzyme according to any of claims 1 to 13.

21. Enzymatic preparation according to claim 19, which contains as a single component an enzyme according to any of claims 1 to 13.

22. Enzymatic preparation according to claim 19, which contains more than one enzyme according to any of claims 1 to 13.

23. Enzymatic preparation according to claim 19, which further comprises at least an enzyme selected from the group formed by cellulases, glucanases, mannanases, chitinases, proteases and/or chitosanases.

24. An antifungal composition that comprises, at least, an enzyme according to any of claims 1 to 13 together with at least a chemical fungicide.

25. Composition according to claim 24, wherein said chemical fungicide is selected from the group formed by a chemical fungicide which affects the membrane, a chemical fungicide which affects cell wall synthesis and their mixtures.

26. Composition according to claim 24 which comprises, additionally, at least, a protein with enzymatic activity for degrading the bacterial cell wall.

27. Use of an enzyme according to any of claims 1 to 33, or of an enzymatic preparation according to any of claims 19 to 23, or of an antifungal composition according to any of claims 24 to 26, to degrade or modify materials which contain β-(1,6)-glucan.

28. Use according to claim 27, wherein said material that contains β-(1,6)-glucan is a microbial cell wall.

29. Use according to claim 27, wherein said enzyme, enzyme preparation or antifungal composition is used for the preparation of protoplasts.

30. Use according to claim 27, wherein said enzyme, enzyme preparation or antifungal composition is used for the preparation of yeast extracts.

31. Use according to claim 27, wherein said enzyme, enzyme preparation or antifungal composition is used in the extraction of mannoproteins.

32. Use according to claim 27, wherein said enzyme, enzyme preparation or antifungal composition is used in the production of wines, musts and juices.

33. Use according to claim 27, wherein said enzyme, enzyme preparation or antifungal composition is used in the elaboration of compositions to remove dental plaque.

34. Use according to claim 27, wherein said enzyme, enzyme preparation or antifungal composition is used in the elaboration of compositions for cleaning teeth and dentures.

35. Use according to claim 27, wherein said enzyme, enzyme preparation or antifungal composition is used in the elaboration of compositions for removing biological films (biofilms) deposited on surfaces.

36. Use according to claim 27, wherein said enzyme, enzyme preparation or antifungal composition is used in the elaboration of compositions for cleaning contact lenses.

37. Use according to claim 27, wherein said enzyme, enzyme preparation or antifungal composition is used in the elimination of fungi on coatings.

38. Use according to claim 27, wherein said enzyme, enzyme preparation or antifungal composition is used for treating fabrics.

39. Use according to claim 27, wherein said enzyme, enzyme preparation or antifungal composition is used in the control of organisms which are pathogens of plants, animals, including man, and contaminants of crops and foodstuffs.

40. Use according to claim 27, wherein said enzyme, enzyme preparation or antifungal composition is used for disinfecting, preventing and/or treating the infection caused by fungal animal pathogens in livestock facilities.

41. Use according to claim 27, wherein said enzyme, enzyme preparation or antifungal composition is used for controlling fungal contamination in a test sample for analysis.
